# EUROPEAN PATENT APPLICATION

(11) **EP 3 662 874 A1**
(43) Date of publication of application: **10.06.2020**
(21) Application number: 18210555.1
(22) Date of filing: 05.12.2018
(51) Int. Cl.: A61F 7/12, A61F 11/00, A61F 13/38

(54) **APPARATUS AND EARPHONES FOR REMOVING EARWAX**

(71) Applicant: Nokia Technologies Oy, 02610 Espoo (FI)
(72) Inventor: AGARWAL, Akshat, Dublin, 15 (IE); O'CONNELL, Diarmuid, Co. Kildare (IE)
(74) Representative: Swindell & Pearson Limited

(57) **Abstract**

Examples of the disclosure relate to an apparatus for removing earwax from the ear canal of a subject. The apparatus comprises an insertion portion which is configured to be inserted into the ear canal of the subject. The apparatus also comprises a heat transfer means. The heat transfer means is configured so that heat can be transferred through the insertion portion to the earwax within the ear canal so as to enable the earwax to be melted. The apparatus also comprises removing means which are configured to remove the melted earwax from the ear canal.

## Description

### TECHNOLOGICAL FIELD

Examples of the present disclosure relate to apparatus and earphones for removing earwax. Some relate to apparatus and earphones for removing earwax by using heat.

### BACKGROUND

Earwax (cerumen) occurs naturally and has the function of cleaning and protecting the ear canal. However, due to the shape of the ear canal earwax can deposit in specific locations. If this waxy build-up is not cleared the earwax can accumulate, sediment and compact into a hardened mass which can be difficult to remove. This accumulation of earwax can lead to numerous issues including discomfort, infection, reduced hearing or even loss of hearing.

### BRIEF SUMMARY

According to various, but not necessarily all, examples of the disclosure there may be provided an apparatus for removing earwax, the apparatus comprising: an insertion portion configured to be inserted into the ear canal of a subject; heat transfer means for transferring heat through, at least part of, the insertion portion to earwax within the ear canal to enable melting of the earwax within the ear canal; and removing means for removing the melted earwax from the ear canal.

The removing means may comprise a wicking structure configured to remove melted earwax from the ear canal by capillary action. The wicking structure may comprise a mesh. The apparatus may comprise sensing means for sensing the amount of earwax that has been absorbed by the wicking structure. The sensing means may be configured to detect deformation of the wicking structure which is caused by the absorption of the melted earwax.

The removing means may comprise a thermally conductive material so that the removing means forms part of the heat transferring means.

An anti-bacterial coating may be provided on at least part of the removing means

The heat transfer means may comprise one or more heat pipes located within the insertion portion.

The apparatus may comprise a heat source configured to provide heat to the heat transfer means.

The heat transfer means may be configured to be coupled to a heat source where the heat source is provided in a different apparatus so as to enable heat from the heat source to be provided to the heat transferring means.

The heat source may comprise one or more electrical components.

The insertion portion may comprise resilient means for pushing the removing means into the ear canal.

The resilient means may comprise at least one spring coupled to the removing means.

The insertion portion may comprise one or more channels configured to enable a solvent to be provided to the earwax.

According to various, but not necessarily all, examples of the disclosure there may be provided an earphone comprising: an apparatus for removing earwax, the apparatus comprising: an insertion portion configured to be inserted into the ear canal of a subject; heat transfer means for transferring heat through, at least part of, the insertion portion to earwax within the ear canal to enable melting of the earwax within the ear canal; and removing means for removing the melted earwax from the ear canal; and at least one sound transducer.

The earphone may comprise processing circuitry wherein the processing circuitry may be thermally coupled to the heat transfer means.

According to various, but not necessarily all, examples of the disclosure there may be provided an apparatus for removing earwax, the apparatus comprising: an insertion portion configured to be inserted into the ear canal of a subject; at least one heat transfer portion configured to transferring heat through, at least part of, the insertion portion to earwax within the ear canal to enable melting of the earwax within the ear canal; and at least one remover configured to remove the melted earwax from the ear canal.

### BRIEF DESCRIPTION

Some example embodiments will now be described with reference to the accompanying drawings in which:
Fig. 1 illustrates an example apparatus;
Figs. 2A to 2C illustrate an example apparatus in use;
Figs. 3A to 3C illustrate another example apparatus in use;
Fig. 4 illustrates an example earphone;
Fig. 5 illustrates an example earphone; and
Fig. 6 illustrates an example apparatus.

### DETAILED DESCRIPTION

Examples of the disclosure relate to an apparatus 101 for removing earwax 213 from the ear canal 211 of a subject. The apparatus 101 comprises an insertion portion 103 which is configured to be inserted into the ear canal 211 of the subject. The apparatus 101 also comprises a heat transfer means 105. The heat transfer means 105 is configured so that heat can be transferred through the insertion portion 103 to the earwax 213 within the ear canal 211 so as to enable the earwax 213 to be melted. The apparatus 101 also comprises removing means 107 which are configured to remove the melted earwax 213 from the ear canal.

The apparatus 101 therefore provides a safe and effective way of removing earwax 213 from an ear canal 211 of a subject. The apparatus 101 could be incorporated into electronic devices such as ear pieces. This may enable the electronic devices to provide dual functionality such as providing audio to a subject while also enabling the earwax to be removed from the subject's ears.

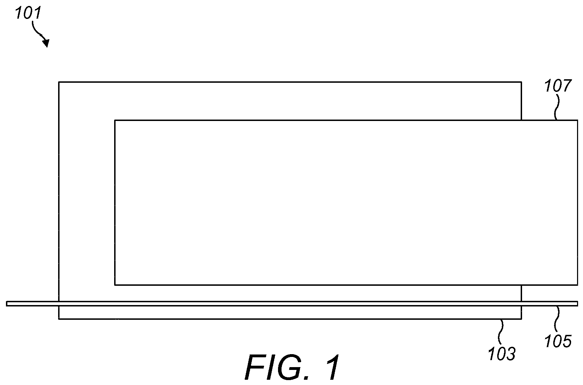

Fig. 1 schematically illustrates an example apparatus 101. The example apparatus 101 comprises an insertion portion 103, heat transfer means 105 and removing means 107. It is to be appreciated that the apparatus 101 could comprise additional features in other examples of the disclosure.

The insertion portion 103 may comprise a portion of the apparatus 101 that is sized and shaped so as to fit into the ear canal 211 of a subject. The insertion portion 103 may have dimensions such that the insertion portion 103 fits snugly into the ear canal 211 of the subject.

The insertion portion 103 may have a length that prevents the insertion portion 103 from reaching the ear drum 215 of the subject when the insertion portion 103 is positioned within the ear canal 211. In some examples the apparatus 101 may be available with insertion portions 103 of different sizes. An apparatus 101 having an insertion portion 103 of the appropriate size can then be selected for the subject based on the size of their ear canal 211.

In some examples the apparatus 101 may also comprise a portion that is not inserted into the ear canal 211 of the subject. The portion that is not inserted into the ear canal 211 may be provided at an end of the apparatus 101 and may be used to remove the apparatus 101 from the ear canal 211.

The heat transfer means 105 may comprise any means that enables heat to be transferred from a heat source to a location within the ear canal 211. The heat transfer means 105 may be positioned within the insertion portion 103 so that, in use, the heat transfer means 105 can make thermal contact with the earwax 213. The thermal contact between the heat transfer means 105 and the earwax 213 enables the melting of the earwax 213 within the ear canal 211.

The heat transfer means 105 extends through at least part of the insertion portion 103 so that heat can be transferred from a location outside of the ear canal 211 to a location within the ear canal 211. In the example shown in Fig. 1 the heat transfer means 105 extends along the entire length of the insertion portion 103. In the example shown in Fig. 1 the heat transfer means 105 extends out of the end of the insertion portion 103. This may facilitate thermal transfer between the heat transfer means 105 and the earwax 213.

The heat transfer means 105 could comprise one or more heat pipes or any other thermal conductor that is configured to transfer heat to the earwax 213 within the ear canal 211. The heat transfer means 105 may comprise any suitable thermally conductive material. For instance the heat transfer means 105 could comprise aluminium, copper or any other suitable material. In some examples the heat transfer means 105 may be insulated, or partially insulated, to prevent heat from leaking out of the heat transfer means 105 along the length of the insertion portion 103.

The heat pipe may comprise an evaporator section, an adiabatic section and a condenser section. The evaporator section is located close to a heat source and is configured to enable heat to be transferred from the heat source to the heat pipe. The condenser section is located close to the section where the heat is to be provided to. In examples of the disclosure the condenser section may be located close to end of the insertion portion 103 so that the heat can be provided to the earwax 213. The adiabatic section is provided between the evaporator section and the condenser section and is configured to reduce the loss of heat as it is transported through the heat pipe.

In some examples the apparatus 101 could comprise a heat source which is configured to provide heat to the heat transfer means 105. The heat source could be provided in a part of the apparatus 101 that is not inserted into the ear canal 211. In other examples the apparatus 101 could comprise means for coupling to another apparatus where the another apparatus comprises a heat source. In such examples the coupling to the another apparatus enables heat to be transferred from the heat source within the another apparatus to the heat transfer means 105. This therefore enables heat from another apparatus to be used to melt earwax 213 within an ear canal 211.

The heat source could comprise any suitable means for generating heat. In some examples the heat source could comprise one or more electrical components. The electrical components could comprise processing circuitry, power sources or any other suitable components. The electrical components could generate heat as an auxiliary effect of performing other functions. The transfer of this heat by the heat transfer means 105 could therefore help with cooling of the electrical components.

The removing means 107 may comprise any means which enables the melted earwax 213 to be removed from the ear canal 211.

In the examples of the disclosure the removing means 107 comprises part of the insertion portion 103 so that, in use, the removing means 107 is positioned within the ear canal 211 of the subject. In examples of the disclosure the removing means 107 is provided at the end of the insertion portion 103 so that when the apparatus 101 is in use the removing means 107 contacts the earwax 213.

In some examples the removing means 107 may be soft and/or deformable. This may make the apparatus 101 comfortable to be inserted into the ear canal 211. This may also help to prevent damage being caused to the ear drum 215 if the apparatus 101 is inserted too far into the ear canal 211.

In some examples the removing means 107 comprises a wicking structure which is configured to remove melted earwax 213 from the ear canal 211 by capillary action. The wicking structure may comprise a mesh or other suitable structure which provides channels that enable the capillary action.

In some examples the wicking structure may be formed using three dimensional printing. This may enable the size of the wicking structure to be controlled so that it fits into the ear canal of a subject. In some examples the use of three dimensional printing may also enable the size of the channels within the wicking structure to be controlled. This will affect the porosity of the wicking structure and how efficiently the wicking structure removes the melted earwax 213 from the ear canal 211.

In some examples the removing means 107 may comprise a thermally conductive material. In such examples this may enable the removing means 107 to form part of the heat transfer means 105. This may enable heat to be transferred through the removing means 107 to the earwax 213 within the canal 211. The thermally conductive material could comprise a metal such as aluminium or any other suitable material.

In some examples an anti-bacterial coating could be provided on at least part of the removing means 107. The anti-bacterial coating may reduce the risk of infections for the user. The anti-bacterial coating may comprise silver, silver alloys or any other suitable material.

In examples of the disclosure both the removing means 107 and the heat transfer means 105 comprise part of the insertion portion 103 so that the removing means 107 and the heat transfer means 105 can be positioned within the ear canal 211 of the subject at the same time. This makes the apparatus 101 simple for a subject to use because they can insert the apparatus 101 into their ear and then leave it until the earwax 213 has been removed by the removing means 107. There is no need for separate steps such as syringing or irrigation in order to remove the earwax 213.

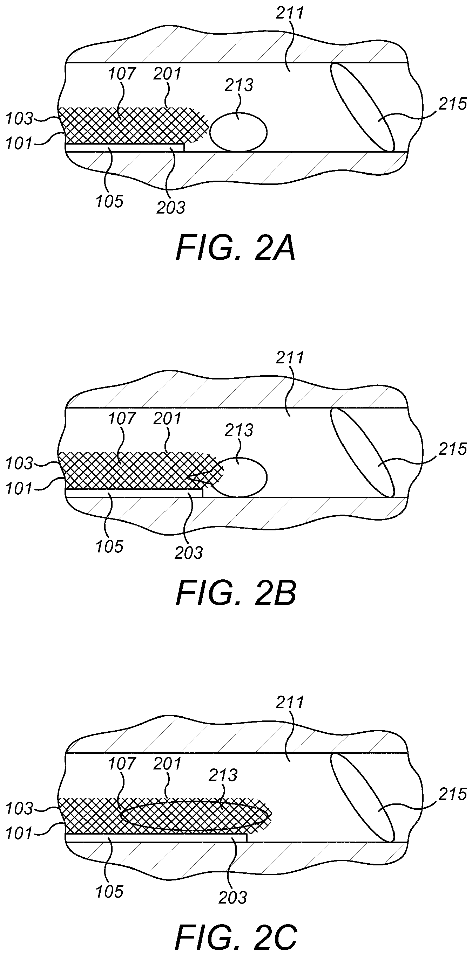

Figs. 2A to 2C illustrate an example apparatus 101 at different stages of use. The apparatus 101 could be an apparatus 101 as shown in Fig. 1 and corresponding reference numerals are used for corresponding features. In the example shown in Figs. 2A to 2C the heat transfer means 105 and the removing means 107 are provided as part of the insertion portion 103.

In the example shown in Figs. 2A to 2C the removing means 107 comprises a wicking structure 201. The wicking structure 201 comprises a mesh which forms channels that enable melted earwax 213 to be transferred via capillary action. The wicking structure 201 extends to the end of the insertion portion 103 so that when the insertion portion 103 is inserted into the ear canal 211 of the subject, the wicking structure 201 makes direct contact with the earwax 213.

In the example shown in Figs. 2A to 2C the heat transfer means 105 comprises a heat pipe 203 that extends along the length of the insertion portion 103. The heat pipe 203 is thermally coupled to the wicking structure 201 to enable heat to be transferred from the heat pipe 203 to the wicking structure 201. In this example the wicking structure 201 may form part of the heat transfer means 105.

Figs. 2A to 2C show an example of the apparatus 101 at different stages of use. In the example shown in Fig. 2A the insertion portion 103 of the apparatus 101 is inserted into the ear canal 211 of the subject. The insertion portion 103 is sized so that the insertion portion 103 extends far enough along the ear canal 22 so that the end of the insertion portion 103 reaches the earwax 213 within the ear canal 211. This ensures that, at least part of, the wicking structure 201 comes into contact with the earwax 213 that is located in front of the ear drum 215.

In this example the wicking structure 201 may comprise a thermally conductive material so that heat can, at least partially, be transferred from the heat pipe 203 to the earwax 213 through the wicking structure 201. In other examples the heat pipe 203 may be positioned within the insertion portion 103 so that the heat pipe 203 contacts the earwax 213 directly. This may enable heat to be transferred directly from the heat pipe 203 to the earwax 213. In such examples the wicking structure 201 need not be comprise a thermally conductive material.

In Fig. 2A the apparatus 101 has just been inserted in the ear canal 211. The insertion portion 103 is pushed far enough into the ear canal 211 so that the wicking structure 201 contacts the earwax 213. As the apparatus 101 has just been inserted no heat has been transferred to the earwax 213 yet so the earwax 213 is still provided in a solid form.

Fig. 2B shows the same apparatus 101 as Fig. 2A after the apparatus 101 has been positioned within the ear canal 211 for some time. During this time heat has been provided through the heat pipe 203 and wicking structure 201 to the earwax 213. This has caused some of the earwax 213 to melt.

Once the earwax 213 has melted the melted earwax 213 is drawn into the wicking structure 201 by capillary action.

Fig. 2C shows the same apparatus 101 as shown in Figs. 2A and 2B after the apparatus 101 has been positioned within the ear canal 211 for a longer period of time. During this time more heat has been provided through the heat pipe 203 and wicking structure 201 to the earwax 213 and this has caused all of the earwax 213 to melt.

In Fig. 2C all of the earwax 213 has been melted and drawn into the wicking structure 201. When the apparatus 101 is removed from the ear canal 211 the melted earwax 213 will be removed with the wicking structure 201. In the example shown in Fig. 2C all of the earwax 213 is melted so that when the apparatus 101 is removed from the ear canal 211 the ear canal 211 is clear of earwax 213.

The time taken for the earwax 213 to be melted and absorbed by the wicking structure 201 may depend on a number of factors such as the amount of earwax 213 within the ear canal 211, the thermal efficiency of the heat transfer means 105, the porosity of the wicking structure 201 and any other suitable factor. In some examples it could take several hours or even days for the earwax 213 to be melted. The insertion portion 103 may therefore be comfortable enough so that it can be positioned within the ear canal 211 for this length of time. The comfort of the insertion portion 103 may be affected by factors such as the size of the insertion portion 103, the softness of the materials used for the insertion portion 103, antibacterial properties of the insertion portion 103 and any other suitable factors.

As the melted earwax 213 is absorbed by the wicking structure 201 it causes deformation of the wicking structure 201. The additional material that is now comprised within the wicking structure 201 may cause bending or bowing of the wicking structure 201. In some examples the apparatus 101 may be configured to enable this deformation to be measured. This measurement can therefore provide an indication of the amount of earwax 213 that has been absorbed by the wicking structure 201.

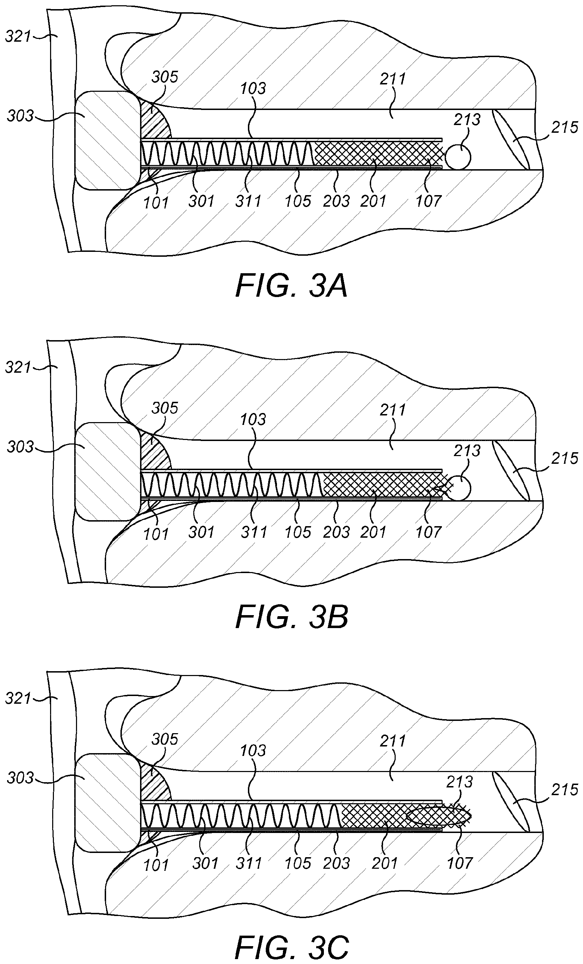

Figs. 3A to 3C illustrate another example apparatus 101 in use. The example apparatus 101 shown in Figs. 3A to 3C also comprises an insertion portion 103, heat transfer means 105 and removing means 107. Corresponding reference numerals are used for corresponding features. In the example shown in Figs. 3A to 3C the apparatus 101 also comprises a resilient means 301, a mount portion 303 and an ear cap 305. In the example shown in Figs. 3A to 3C the heat transfer means 105 comprises a heat pipe 205 and the removing means 107 comprises as wicking structure 201 as shown in Figs. 2A to 2C. It is to be appreciated that other types and/or configurations of heat transfer means 105 and removing means 107 could be used in other examples of the disclosure.

The resilient means 301 provides means for pushing the removing means 107 into the ear canal 211. The resilient means 301 may comprise any means which provides a force that pushes the removing means 107 into the ear canal 211. In the example shown in Figs. 3A to 3C the resilient means 301 comprises a spring 311. The spring 311 is provided within the insertion portion 103. The spring 311 is coupled to the removing means 107. In the example shown in Figs. 3A to 3C the spring 311 is coupled to the wicking structure 201 of the removing means 107 so that movement of the spring 311 causes movement of the wicking structure 201.

In the examples shown in Figs. 3A to 3C the spring 311 and the wicking structure 201 are configured so that they can move relative to other components of the insertion portion 103. In the examples shown the spring 311 can be compressed or extended which allows for movement along the length of the insertion portion 103. As the wicking structure 201 is coupled to the spring 311 the compression or extension of the spring 311 causes movement of the wicking structure 201 along the length of the ear canal 211. This may enable movement of the wicking structure 201 relative to other components of the insertion portion 103 such as the heat pipe 205.

The apparatus 101 also comprises a mount portion 303. The mount portion 303 is provided on the opposite end of the insertion portion 103 to the removing means 107. The mount portion 303 comprises a portion that is not inserted into the ear canal 211 of the subject. The mount portion 303 is sized and shaped so that it does not fit into the ear canal 211.

As shown in Figs. 3A to 3C, when the apparatus 101 is in use the mount portion 303 is located within the outer ear 321. The mount portion 303 may therefore prevent the insertion portion 103 from being inserted too far into the ear canal 211. The mount portion 303 may also be used to insert and remove the apparatus 201 from the subject's ear. For instance, the subject can grip the mount portion 303 to insert or remove the apparatus 101 as needed.

The mount portion 303 may comprise any suitable material. In some examples the mount portion 303 may comprise a rigid material such as a plastic.

In some examples the mount portion 303 may comprise additional components that are not shown in Figs. 3A to 3C. For instance the mount portion 303 may comprise a heat source or means for coupling the heat transfer means 105 to an external heat source. In some examples the mount portion 303 could comprise electronic components. The electronic components could provide a function to the subject. For example the electronic components could provide audio and/or process audio for rendering to the subject. In such examples the electronic components could generate heat which could be provided to the heat transfer means 105.

In the example shown in Figs. 3A to 3C the apparatus 101 also comprises an ear cap 305. The ear cap 305 is coupled to the mount portion 303. The ear cap 305 is coupled to the mount portion 303 on the same side as the insertion portion 103 so that when the apparatus 101 is in use the ear cap 305 is positioned in the entrance of the ear canal 211.

The ear cap 305 may comprise a flexible material. The ear cap 305 may provide a seal, or at least a partial seal, of the end of the ear canal 211. The ear cap 305 may help the apparatus 101 to fit comfortably into the ear of the subject. The ear cap 305 may help the apparatus 101 to fit into different sized and shaped ears.

In Fig. 3A the apparatus 101 has just been inserted in the ear canal 211. The insertion portion 103 is pushed far enough into the ear canal 211 so that the wicking structure 201 contacts the earwax 213. As the apparatus 101 has just been inserted no heat has been transferred to the earwax 213 yet so the earwax 213 is still provided in a solid form.

The solid earwax 213 provides a resistive force to the wicking structure 201 and the resilient means 301. The mount portion 303 is pushed into the ear so that the ear cap 305 fits tightly into the ear. This causes compression of the spring 311. In the configuration shown in Fig. 3A the spring 311 is compressed so that the wicking structure 201 does not extend past the end of the heat pipe 105.

Fig. 3B shows the same apparatus 101 as Fig. 3A after the apparatus 101 has been positioned within the ear canal 211 for some time. During this time heat has been provided through the heat pipe 203 and wicking structure 201 to the earwax 213. This has caused some of the earwax 213 to melt.

Once the earwax 213 has melted the melted earwax 213 is drawn into the wicking structure 201 by capillary action. This reduces the volume of earwax 213 within the ear canal 211 and so decreases the resistive force applied by the earwax 213. This causes some decompression of the spring 311 which pushes the wicking structure 201 further along the length of the ear canal 211. In the example shown in Fig. 3B the wicking structure 201 now extends past the end of the heat pipe 205. This movement of the wicking structure 201 ensures that, as the earwax 213 is melted, at least some of the earwax 213 remains in contact with the wicking structure 201.

Fig. 3C shows the same apparatus 101 as shown in Figs. 3A and 3B after the apparatus 101 has been positioned within the ear canal 211 for a longer period of time. During this time more heat has been provided through the heat pipe 203 and wicking structure 201 to the earwax 213 and this has caused all of the earwax 213 to melt.

In Fig. 3C all of the earwax 213 has been melted and drawn into the wicking structure 201. When the apparatus 101 is removed from the ear canal 211 the melted earwax 213 will be removed with the wicking structure 201. In the example shown in Fig. 3C all of the earwax 213 is melted so that when the apparatus 101 is removed from the ear canal 211 the ear canal 211 is clear of earwax 213.

In the example shown in Fig. 3C all of the earwax 213 is now absorbed within the wicking structure 201. In this case there is no more solid earwax 213 to provide a resistive force to the wicking structure 201 and spring 311. This causes some decompression of the spring 311 which pushes the wicking structure 201 even further along the length of the ear canal 211. In this configuration the wicking structure 201 extends further past the end of the heat pipe 203.

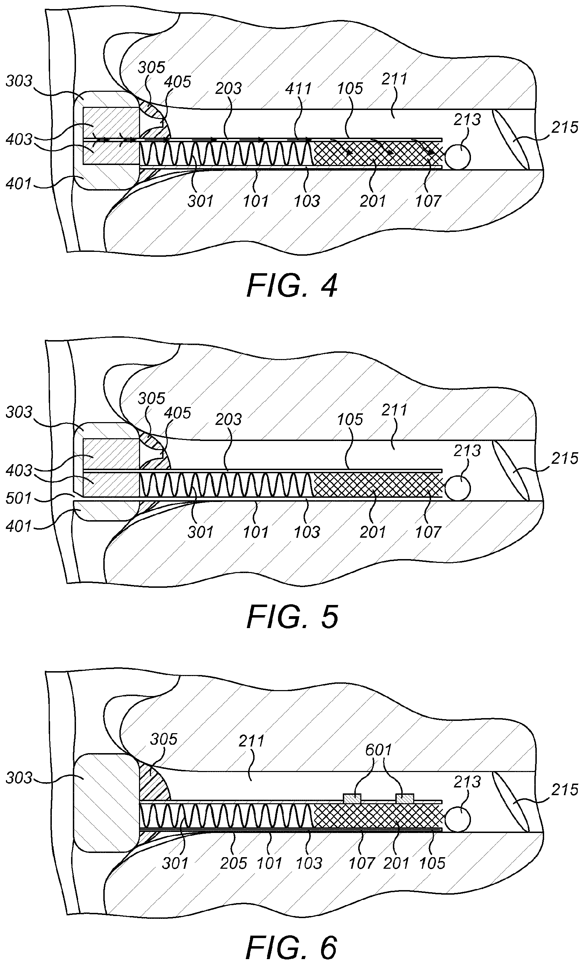

Fig. 4 illustrates an earphone 401 comprising another example apparatus 101. The earphone 401 is configured to provide acoustic signals to the subject. In the example shown in Fig. 4 the earphone 401 is an inner earphone or an ear bud. Other types of earphone 401 could be used in other examples of the disclosure.

The example apparatus 101 shown in Fig. 4 also comprises an insertion portion 103, heat transfer means 105, removing means 107, resilient means 311, a mount portion 303 and an ear cap 305. Corresponding reference numerals are used for corresponding features. In the example shown in Fig. 4 the heat transfer means 105 comprises a heat pipe 205 and the removing means 107 comprises as wicking structure 201 as shown in Figs. 2A to 3C. It is to be appreciated that other types and/or configurations of heat transfer means 105 and removing means 107 could be used in other examples of the disclosure.

The earphone 401 shown in Fig. 4 comprises electronic components 403 and an earphone output 405 in addition to the apparatus 101.

In the example of Fig. 4 the electronic components 403 are, at least partially, provided in the mount portion 303 of the apparatus 101. The electronic components could comprise sound transducers, processing circuitry, power sources and/or any other suitable electronic components 403.

The earphone output 405 is provided on the mount portion 303. The earphone phone output 405 comprises an acoustic channel through which an acoustic signal can travel. This enables sound to be provided from the earphone 401 to the ear canal 211.

The earphone output 405 is provided on the same side of the mount portion 303 as the insertion portion 103 so that, in use the earphone output 405 directs the acoustic signals towards the ear drum 215.

In the examples shown in Fig. 4 the heat transfer means 105 is thermally coupled to the electronic components 403. The electronic components 403 may generate heat as they are performing functions relating to processing and/or audio rendering. The heat transfer means 105 enables this heat to be removed from the electronic components 403 and provided to the earwax 213 within the ear canal 211.

In the example shown in Fig. 4 the arrows 411 show the heat flow within the apparatus 101. This shows that heat travels from the electronic components 403, through the heat pipe 201 and wicking structure 201 to the earwax 213.

In the example of Fig. 4 the apparatus 101 is therefore configured to take unwanted heat from the electronic components 403 and use this to melt the earwax 213. This provides a device with dual functionality of providing acoustic signals and also removing earwax 213. The transfer of the heat away from the electronic components 403 also helps to cool the electronic components 403.

In other examples instead of electronic components 403 the apparatus 101 could comprise a heat source which is configured just to provide heat for the heat transfer means 105.

In other examples the heat source could be provided externally to the apparatus 101. In such examples the apparatus 101 could comprise coupling means which could be configured to couple the apparatus 101 to another apparatus which comprises a heat source. The another apparatus could be a head set such as a virtual reality headset or an augmented reality headset. Such apparatus may comprise processing circuitry and/or other components that generate heat that can be coupled to the heat transfer means 105. For example a pair of augmented or virtual reality glasses could comprise processing components in the arm of the glasses. When the glasses are in use these components would be positioned close to the subject's ear and could be thermally coupled to the heat transfer means 105.

Fig. 5 illustrates an example earphone 401 comprising an apparatus 101. The earphone 401 and apparatus 101 shown in Fig. 5 are similar to the apparatus 101 and earphone 401 shown in Fig. 4 and corresponding reference numerals are used for corresponding features.

In the example earphone 401 shown in Fig. 5 the apparatus 101 also comprises a channel 501 which is configured to enable a solvent to be provided to the earwax 213. In the example of Fig. 5 one channel 501 is shown, however it is to be appreciated that more than one channel 501 could be provided in other examples of the disclosure.

The channel 501 extends along the length of the insertion portion 103. The channel 501 has an opening at the end of the insertion portion 103 to enable the solvent to flow from the channel 501 to the earwax 231. In the example shown in Fig. 5 the channel 501 also extends through the mount portion 303 to the insertion portion 103. This enables ear drops or other solvents to be provided to the earwax 213 while the apparatus 101 is positioned within the subject's ear. The use of the solvents may soften the earwax 213 so that the earwax 213 melts more easily when the heat is provided by the heat transfer means 105. This may reduce the length of time that the apparatus 101 needs to be positioned within the subject's ear.

It is to be appreciated that the channel 501 could also be provided within the apparatus 101 which do not form part of an earphone 401. For instance one or more channels 501 could be provided within apparatus 101 such as the apparatus 101 shown in Figs. 2A to 3C.

Fig. 6 illustrates another example apparatus 101. The example apparatus 101 is similar to that shown in Figs. 3A to 3C. In the example of Fig. 6 the apparatus 1 also comprises one or more sensing means 601.

The sensing means 601 may comprise any means that is configured to sense the amount of earwax 213 that has been absorbed by the wicking structure 201.

In the example shown in Fig. 6 the sensing means 601 are configured to detect deformation of the wicking structure 201 which is caused by the absorption of the melted earwax 213. In the example shown in Fig. 6 the sensing means 601 comprises one or more strain sensors located within the insertion portion 103. The strain sensors maybe located within the walls of the insertion portion 103 adjacent to the wicking structure 201. When the wicking structure 201 absorbs melted earwax 213 the extra material absorbed by the wicking structure 201 causes the wicking structure 201 to expand. The expansion of the wicking structure 201 causes strain to be applied to the insertion portion 103. The strain sensors 601 could be configured to measure this strain and use this measurement to determine how much earwax 213 has been absorbed by the wicking structure 201.

In some examples the apparatus 101 comprises a plurality of strain sensors 601 where the strain sensors 601 are provided at different locations along the length of the insertion portion 103. When different strain sensors 601 detect a strain this provides an indication of how far along the wicking structure 201 the earwax 213 has been absorbed. This also provides an indication of how much earwax 213 has been melted.

In other examples the sensing means 601 could comprise means for sensing the position of the spring 311 and/or the load applied to the spring 311. This could sense how much the spring 311 has been compressed and/or how much the spring 311 has moved. This provides an indication of the resistive force applied by the solid earwax 213. This also provides an indication of how much earwax 213 has been melted and absorbed by the wicking structure 201.

In some examples the apparatus 101 could comprise means for both sensing the deformation of the wicking structure 201 and for sensing the forces applied to the spring 311. The information from these different types of sensing means 601 could be used in combination to determine how much earwax 213 has been absorbed. In some examples this information could be used to determine whether or not all of the earwax 213 has been melted and absorbed by the wicking structure 201. This information could be used to provide an indication of when the apparatus 101 should be removed from the ear canal 211.

In some examples the information from the sensing means 601 could be provided to processing circuitry. The processing circuitry could be configured to determine the amount of earwax removed and/or other information that may be useful for the subject. In some examples the processing circuitry could be provided in the mount portion 303 of the apparatus 101. In other examples the processing circuitry could be provided in another apparatus.

In some examples the apparatus 101 could also comprise an output device which enables information obtained from the sensing means 601 to be provided to the subject. For instance, where the apparatus 101 comprises part of an earphone 401 the earphone 401 may be configured to provide an audible output to the subject. In other examples the apparatus 101 could be coupled to another device such as a mobile device. The mobile device could comprise an application which links to the apparatus 101 and enables information from the sensing means 601 to be provided to the subject or other user, such as a medical professional.

It is to be appreciated that the apparatus 101 may be provided in configurations that differ to those shown in the Figs. For instance Figs. 4 to 6 all show the use of a resilient means 301, a mount portion 303 and an ear cap 305 however one or more of these components could be omitted in some examples of the disclosure.

Therefore examples of the disclosure provide an apparatus 101 that enables earwax 213 to be removed from an ear canal 211 by applying heat directly to the earwax 213. The apparatus 101 could also provide dual functionality in that it may be part of an earphone 401 or other suitable device and/or may assist with the cooling of electronic components 403.

In this description the term coupled means operationally coupled and any number or combination of intervening elements can exist between coupled components including no intervening elements.

The term 'comprise' is used in this document with an inclusive not an exclusive meaning. That is any reference to X comprising Y indicates that X may comprise only one Y or may comprise more than one Y. If it is intended to use 'comprise' with an exclusive meaning then it will be made clear in the context by referring to 'comprising only one...' or by using 'consisting'.

In this description, reference has been made to various examples. The description of features or functions in relation to an example indicates that those features or functions are present in that example. The use of the term 'example' or 'for example' or 'can' or 'may' in the text denotes, whether explicitly stated or not, that such features or functions are present in at least the described example, whether described as an example or not, and that they can be, but are not necessarily, present in some of or all other examples. Thus 'example', 'for example', 'can' or 'may' refers to a particular instance in a class of examples. A property of the instance can be a property of only that instance or a property of the class or a property of a subclass of the class that includes some but not all of the instances in the class. It is therefore implicitly disclosed that a feature described with reference to one example but not with reference to another example, can where possible be used in that other example as part of a working combination but does not necessarily have to be used in that other example.

Although embodiments have been described in the preceding paragraphs with reference to various examples, it should be appreciated that modifications to the examples given can be made without departing from the scope of the claims.

Features described in the preceding description may be used in combinations other than the combinations explicitly described above.

Although functions have been described with reference to certain features, those functions may be performable by other features whether described or not.

Although features have been described with reference to certain embodiments, those features may also be present in other embodiments whether described or not.

The term 'a' or 'the' is used in this document with an inclusive not an exclusive meaning. That is any reference to X comprising a/the Y indicates that X may comprise only one Y or may comprise more than one Y unless the context clearly indicates the contrary. If it is intended to use 'a' or 'the' with an exclusive meaning then it will be made clear in the context. In some circumstances the use of 'at least one' or 'one or more' may be used to emphasis an inclusive meaning but the absence of these terms should not be taken to infer and exclusive meaning.

The presence of a feature (or combination of features) in a claim is a reference to that feature or (combination of features) itself and also to features that achieve substantially the same technical effect (equivalent features). The equivalent features include, for example, features that are variants and achieve substantially the same result in substantially the same way. The equivalent features include, for example, features that perform substantially the same function, in substantially the same way to achieve substantially the same result.

In this description, reference has been made to various examples using adjectives or adjectival phrases to describe characteristics of the examples. Such a description of a characteristic in relation to an example indicates that the characteristic is present in some examples exactly as described and is present in other examples substantially as described.

Whilst endeavoring in the foregoing specification to draw attention to those features believed to be of importance it should be understood that the Applicant may seek protection via the claims in respect of any patentable feature or combination of features hereinbefore referred to and/or shown in the drawings whether or not emphasis has been placed thereon.

## Claims

1. An apparatus for removing earwax, the apparatus comprising:
an insertion portion configured to be inserted into the ear canal of a subject;
heat transfer means for transferring heat through, at least part of, the insertion portion to earwax within the ear canal to enable melting of the earwax within the ear canal; and
removing means for removing the melted earwax from the ear canal.

2. An apparatus as claimed in claim 1 wherein the removing means comprises a wicking structure configured to remove melted earwax from the ear canal by capillary action.

3. An apparatus as claimed in claim 2 wherein the wicking structure comprises a mesh.

4. An apparatus as claimed in any of claims 2 to 3 comprising sensing means for sensing the amount of earwax that has been absorbed by the wicking structure.

5. An apparatus as claimed in claim 4 wherein the sensing means are configured to detect deformation of the wicking structure which is caused by the absorption of the melted earwax.

6. An apparatus as claimed in any preceding claim wherein the removing means comprises a thermally conductive material so that the removing means forms part of the heat transferring means.

7. An apparatus as claimed in any preceding claim wherein an anti-bacterial coating is provided on at least part of the removing means

8. An apparatus as claimed in any preceding claim wherein the heat transfer means comprises one or more heat pipes located within the insertion portion.

9. An apparatus as claimed in any preceding claim wherein the apparatus comprises a heat source configured to provide heat to the heat transfer means.

10. An apparatus as claimed in any of claims 1 to 8 wherein the heat transfer means is configured to be coupled to a heat source where the heat source is provided in a different apparatus so as to enable heat from the heat source to be provided to the heat transferring means.

11. An apparatus as claimed in any of claims 9 to 10 wherein the heat source comprises one or more electrical components.

12. An apparatus as claimed in any preceding claim wherein the insertion portion comprises resilient means for pushing the removing means into the ear canal.

13. An apparatus as claimed in claim 12 wherein the resilient means comprises at least one spring coupled to the removing means.

14. An apparatus as claimed in any preceding claim wherein the insertion portion comprises one or more channels configured to enable a solvent to be provided to the earwax.

15. An earphone comprising:
an apparatus for removing earwax, the apparatus comprising: an insertion portion configured to be inserted into the ear canal of a subject; heat transfer means for transferring heat through, at least part of, the insertion portion to earwax within the ear canal to enable melting of the earwax within the ear canal; and removing means for removing the melted earwax from the ear canal; and
at least one sound transducer.
